# EUROPÄISCHE PATENTSCHRIFT

(11) **EP 1 009 999 B1**
(45) Veröffentlichungstag und Bekanntmachung des Hinweises auf die Patenterteilung: **14.11.2001**
(21) Anmeldenummer: 98946404.5
(22) Anmeldetag: 21.08.1998
(51) Int. Cl.: G01N 21/35

(54) **VERFAHREN UND VORRICHTUNG ZUR IDENTIFIZIERUNG VON WIRKSTOFFEN**
METHOD AND DEVICE FOR IDENTIFYING ACTIVE SUBSTANCES
PROCEDE ET DISPOSITIF D'IDENTIFICATION DE PRINCIPES ACTIFS

(30) Priorität: 04.09.1997 DE 19738566
(43) Veröffentlichungstag der Anmeldung: 21.06.2000
(73) Patentinhaber: Forschungszentrum Karlsruhe GmbH, 76133 Karlsruhe (DE); BASF AKTIENGESELLSCHAFT, 67056 Ludwigshafen (DE)
(72) Erfinder: MOSS, David, D-76337 Waldbronn (DE); PLATSCH, Herbert, D-64297 Darmstadt (DE); FÜCHSLE, Kathrin, D-76227 Karlsruhe (DE); MASUCH, Ralf, D-76135 Karlsruhe (DE)
(74) Vertreter: Rückert, Friedrich, Dr.
(86) Internationale Anmeldenummer: EP9805328
(87) Internationale Veröffentlichungsnummer: WO9912017

(56) Entgegenhaltungen:
- WO-A-85/04478
- WO-A-96/18096
- US-A- 5 339 255

## Beschreibung

Die Erfindung betrifft ein Verfahren zur Identifizierung von Wirkstoffen gemäß dem ersten Patentanspruch und eine Vorrichtung zur Durchführung des Verfahrens gemäß dem neunten Patentanspruch.

M. J. Swamy, T. Heimburg und D. Marsh in "Fourier-Transform Infrared Spectroscopic Studies on Avidin Secondary Structure and Complexation with Biotin and Biotin-Lipid Assemblies", Biophysical Journal Vol. 71 (1996) 840-847 beschreiben Arbeiten zur Aufklärung der Struktur von Komplexen des Proteins Avidin mit Biotin und Biotin-Lipid durch Fourier-Transform-Infrarot-Spektroskopie (FTIR). Hierzu wurden zuerst FTIR-Spektren des Avidin in schwerem Wasser (D₂O) aufgenommen. Anschließend wurde Avidin mit Biotin oder Biotin-Lipid mit gepuffertem D₂O als Lösungsmittel vermischt und mehrere Stunden bei Raumtemperatur aufbewahrt, wodurch offensichtlich eine möglichst hohe Ausbeute des entstehenden Avidin-Komplexes erzielt werden sollte. Von dem Komplex wurden wiederum FTIR-Spektren aufgenommen. Aus den Spektren des Avidins und den Spektren des Avidin-Komplexes wurden Differenzspektren gebildet. Da sich diese Arbeit nur mit der Struktur der Avidin-Komplexe befaßt, werden keine zeitabhängigen Spektren aufgenommen. Die beschriebenen FTIR-Spektren spiegeln in allen Fällen Gleichgewichtszustände wieder. Es werden nicht die Schwingungsspektren von gebundenem normalem Wasserstoff, sondern die - infolge der höheren Masse verschobenen - Schwingungsspektren von gebundenem Deuterium aufgenommen. Infolgedessen kann eine vergleichsweise dicke Küvette (50 µm) eingesetzt werden. Die Frage, ob eine chemische Verbindung, z. B. ein Protein, an einer Koordinationsstelle mit einem bestimmten Liganden einen Komplex zu bilden vermag, spielt in dieser Arbeit keine Rolle, da die Tatsache der Komplexbildung von Avidin mit Biotin bereits bekannt war.

FTIR-Untersuchungen zur Strukturbestimmung von Protein-Komplexen werden außerdem bei M. Gonzales et al: "Interaction of Biotin with Streptavidin", The Journal of Biological Chemistry Vol. 272, No. 17 (1997) 11288-11294 beschrieben. Die Spektren wurden sowohl mit einem H₂O- als auch mit einem D₂O-Puffer aufgenommen, wobei die Schichtdicke im Falle von H₂O 6 µm und im Falle von D₂O 50 µm betrug. Ziel der Arbeit ist die Bestimmung der thermischen Stabilität von Biotin und des Biotin-Streptavidin-Komplexes. Die thermische Denaturierung wird in zeitlich aufeinanderfolgenden Spektren dargestellt. Die Bildung der Komplexe wird dagegen spektrometrisch nicht verfolgt.

In D. Moss, E. Nabedryk, J. Breton und W. Mäntele: "Redox-linked conformational changes in proteins detected by a combination of infrared spectroscopy and protein electrochemistry - Evaluation of the technique with cytochrome c" Eur. J. Biochem. 187, 565-572 (1990) wird über eine elektrochemische Reduktion und eine anschließende erneute Oxidation des Proteins Cytochrom c berichtet. Cytochrom c wird in einer Schichtdicke von 10 bis 15 µm vorgelegt, um die IR-Absorption von Wasser im mittleren Infrarotbereich auszuschließen. Die Reduktion und anschließende erneute Oxidation wurden mit Hilfe der FTIR-Spektroskopie nachgewiesen. Differenzspektren des reduzierten und erneut oxidierten Zustandes sind dargestellt. Da die Küvette nur Cytochrom c enthielt, konnte über die Bildung von Proteinkomplexen keine Aussage gemacht werden.

Die Veröffentlichung von A. J. White, K. Drabble und C. W. Wharton: "A stopped-flow apparatus for infrared spectroscopy of aqueous solutions", Biochem. J. (1995) 306, 843-849 beschreibt eine Apparatur zur Durchführung des sogenannten "stopped-flow"-Verfahrens, in der die Reagentien mit Spritzen in eine Küvette gespritzt und vermischt werden. HPLC-Ventile haben sich nach Aussage der Autoren wegen des erforderlichen hohen Drucks und der hohen Viskosität von Peptiden nicht bewährt. Mit dieser Apparatur wurden FTIR-Spektren im zeitlichen Bereich von 6,25 Sekunden bis 966 Sekunden nach der Vermischung von 12C=O- und 13C=O-Cinnamoyl-Chymotrypsin mit einem Deacylierungsmittel in einem D₂O-Puffer aufgenommen, wobei die optische Schichtdicke 50 µm beträgt. Aus den Spektren von 12C=O- und 13C=O-Cinnamoyl-Chymotrypsin werden Differenzspektren gebildet. In den "Conclusions" findet sich die Feststellung, daß der Aufbau einer "stopped-flow"-IR-Transmissionsküvette, die die Verwendung von (nicht deuteriertem) Wasser zuläßt, nicht möglich ist, weil die starke Absorption von Wasser bei 1640 cm⁻¹ eine Schichtdicke von 5 um verlangt (in der Arbeit werden fälschlich 5 mm angegeben).

Eine eingehende Beschreibung des "stopped-flow"-Verfahrens findet sich bei Q. H. Gibson und L. Milnes: "Apparatus for Rapid and Sensitive Spectrophotometry", Biochem. J. (1964) 91, 161-171.

Ein genereller Nachteil der "stopped-flow"-Methoden ist im hohen Totvolumen der Apparaturen infolge der Verwendung von Spritzen zu sehen. Massenscreening-Verfahren können deshalb mit solchen Apparaturen nicht durchgeführt werden, insbesondere weil die Mikrotiterplatte mit 96 Vertiefungen von je 400 µl die Standardvorlage für automatisierte Verfahren ist; siehe hierzu J. R. Broach und J. Thorner: "High-troughput screening for drug discovery", Nature Vol. 384 Supp. 7. November 1996, die einen Überblick über Massenscreening-Verfahren vermitteln. Broach und Thorner zitieren im Hinblick auf die Feststellung der Bindungsfähigkeit eines Liganden am Rezeptor eines Peptids ein Verfahren, wobei Eu²⁺ am Ligand und Allophycocyanin am Rezeptor kovalent gebunden werden. Durch die Bildung eines Rezeptor-Ligand-Komplexes kommt Eu²⁺ sehr nahe an Allophycocyanin heran, wodurch sich ein als Fluoreszenzsignal nachweisbarer Energietransfer ergibt.

Der Erfindung liegt die Aufgabe zugrunde, ein Verfahren zur Identifizierung von Wirkstoffen zu entwickeln, das den Nachweis der Bildung von Komplexen zwischen Reaktanden in einem möglichst geringen Volumen kostengünstig flexibel und schnell mit reproduzierbaren Ergebnissen ermöglicht. Darüberhinaus soll sich das Verfahren für eine Automatisierung eignen. Weiterhin soll eine Vorrichtung zur Durchführung des Verfahrens vorgeschlagen werden.

Die Aufgabe wird durch das im ersten Patentanspruch beschriebene Verfahren und durch die im neunten Patentanspruch beschriebene Vorrichtung gelöst. In den abhängigen Ansprüchen werden bevorzugte Ausgestaltungen des Verfahrens angegeben.

Erfindungsgemäß werden die Wirkstoffe identifiziert, indem die Bindungsfähigkeit eines Reaktanden, beispielsweise eines Liganden, an mindestens einem weiteren Reaktanden, beispielsweise einem Protein, geprüft wird. Aus den Reaktanden wird eine Mischung hergestellt, von der zu mindestens zwei verschiedenen Zeitpunkten ein IR- oder FTIR-Spektrum aufgenommen wird. Die Messung der Liganden kann in wäßriger Lösung erfolgen, wobei die herkömmliche Verwendung von deuterierten Lösungen, beispielsweise deuteriertes Wasser oder deuterierter Puffer, nicht zwingend erforderlich ist. Je nach der Viskosität und den physikalisch-chemischen Eigenschaften der Reaktanden ist gegebenenfalls der Einsatz eines anderen oder weiteren Lösungsmittels angezeigt; auf den Einsatz deuterierter Puffer oder deuterierter Lösungsmittel kann verzichtet werden.

Die Mischung kann entsprechend dem zitierten Stand der Technik hergestellt werden. Bevorzugt wird jedoch die Verwendung von Hochdruckpumpen (bis ca. 400 bar) und der aus der HPLC-Technik bekannten Probenschleifenventile.

Die Mischung soll vorzugsweise in einer Schichtdicke von 1 bis 25 µm, besonders bevorzugt von 8 bis 15 µm, vorgelegt werden. Zweckmäßigerweise wird die Mischung auf dem Weg zu einer und/oder in einer IR-Küvette mit einer entsprechenden optischen Dicke hergestellt.

In der Regel wird man bemüht sein, eine vollständige Mischung aus der organischen Verbindung und dem Reagenz herzustellen. Da Reaktionen von organischen Verbindungen meist langsam ablaufen, ist die Zeit, die zur Herstellung einer optimalen Mischung und zur Aufnahme des ersten IR- oder FTIR-Spektrums benötigt wird, im allgemeinen ausreichend kurz. Ist jedoch die Reaktionsgeschwindigkeit zwischen den Reaktanden hoch, kann es sich empfehlen, das erste IR- oder FTIR-Spektrum mit einer unvollständigen Mischung aufzunehmen, um zu verhindern, daß sich bis zu diesem Zeitpunkt bereits ein wesentlicher Reaktionsumsatz einstellt.

Bei der Aufnahme des ersten IR- oder FTIR-Spektrums müssen die Reaktanden mindestens teilweise noch in nicht umgesetzter Form vorliegen, so daß die Bildung des Komplexes durch das zweite oder durch weitere IR- oder FTIR-Spektren erfaßt werden kann. Idealerweise soll die Reaktion der Reaktanden nach der Mischung noch nicht eingesetzt haben. Dies ist bei sehr schnellen Reaktionen durch die Mischung vor und/oder auf dem Weg zur IR-Küvette nicht möglich, so daß ein Teil der Reaktanden schon miteinander reagiert hat. Beim erfindungsgemäßen Verfahren stört eine teilweise Reaktion der Reaktanden nicht, solange noch genügend Reaktanden miteinander reagieren können und ein Meßsignal erhalten werden kann. Liegt bei der Aufnahme des ersten IR- oder FTIR-Spektrums ein zu geringer Anteil der Reaktanden in nicht umgesetzter Form vor, so können nur Spektren aufgenommen werden, die keine ausreichenden Unterschiede mehr aufweisen. Die Differenzspektren zeigen in einem solchen Fall eine Nullinie.

Vorzugsweise wird unverzüglich nach der Herstellung der Mischung ein erstes FTIR-Spektrum aufgenommen (Zeitpunkt t₀). "Unverzüglich" bedeutet, daß das Spektrum so schnell wie technisch möglich aufgenommen wird. Da die Reaktionsgeschwindigkeit unmittelbar, nachdem die Reaktanden in Kontakt miteinander gebracht werden, am höchsten ist, ist es von wesentlicher Bedeutung für die Schnelligkeit und Genauigkeit des Verfahrens, wenn bei der Aufnahme des ersten Spektrums die Reaktionsgeschwindigkeit noch ausreichend hoch ist und wenn erst anschließend der Hauptteil der Reaktanden reagiert. Vor allem bei langsameren Reaktionen kann bis zur Aufnahme des ersten Spektrums noch einige Zeit gewartet werden, soweit die Reaktionsgeschwindigkeit der Reaktanden anschließend noch ausreichend groß ist, um sie meßtechnisch zu erfassen. Vorteilhafterweise wird jedoch das erste Spektrum innerhalb von einer bis 1000 Millisekunden nach der Mischung der Reaktanden aufgenommen.

Beim erfindungsgemäßen Verfahren können zur Identifizierung des Wirkstoffs Differenzspektren von zwei zu beliebigen Zeitpunkten aufgenommenen Spektren gebildet werden. Vorzugsweise wird das Differenzspektrum mit dem unmittelbar nach dem Vermischen der Reaktanden aufgenommenen Spektrum (Meßzeitpunkt t₀) gebildet. Als zweites Spektrum wird bevorzugt ein Spektrum, das einen großen zeitlichen Abstand zu diesem Spektrum aufweist, gewählt. Werden mehr als_zwei Reaktanden in der Messung verwendet, so wird die Reaktion vorzugsweise durch Zugabe desjenigen Reaktanden gestartet, der zu der zu untersuchenden Komplexbildung führt.

Als Reaktanden werden solche Stoffe eingesetzt, deren pharmazeutische oder Pflanzenschutzwirkung vermutet wird und die näher untersucht werden sollen. Reaktanden können beispielsweise potentielle Arzneimittel, potentielle Herbizide, Fungizide oder Insektizide sein, die in der Lage sind, Komplexe zu bilden.

Vorzugsweise sind unter den Wirkstoffen im erfindungsgemäßen Verfahren Stoffe zu verstehen, die eine physiologische Wirkung im pflanzlichen, tierischen oder menschlichen Körper entfalten, wie z. B. Hormone, Vitamine, Enzyme, Pharmaka oder Schädlingsbekämpfungsmittel. Als Wirkstoffe seinen Reaktanden wie Proteine, zum Beispiel Enzyme wie ECE oder ACE, Rezeptoren wie Glutamatrezeptoren, Antikörper, Proteininhibitoren wie PAI, Mediatoren, beispielsweise Interferone wie Gamma-Interferon, Interleukine wie Interleukin-2 oder Interleukin-6, Transcriptionsfaktoren wie Spl, Regulatorproteine, Translokatoren oder Chaperone beispielhaft genannt.

Auch niedermolekulare Substanzen mit einem mittleren Molekulargewicht in einem bevorzugten Molekulargewichtsbereich von 100 bis 10000 Dalton (= d), besonders bevorzugt in einem Bereich von 100 bis 1000 d seien hier genannt. Unter niedermolekularen Substanzen sind organische chemische Verbindungen zu verstehen, die beispielsweise gegebenenfalls substituierte aliphatische oder aromatische Heterocyclen, Aromaten, gesättigte oder ungesättigte Aliphaten, Amine, Ketone, Thioketone, Alkohole, Thiole, Ester, Amide, Ether, Thioether, Nitrile, Isonitrile, Aldehyde oder deren Derivate enthalten.

Auch Wirkstoffe, die über die Freisetzung eines Liganden, der als Reaktand schließlich mit dem oder den weiteren Reaktanden einen Komplex bildet, können mit dem erfindungsgemäßen Verfahren erfaßt werden.

Beim erfindungsgemäßen Verfahren ist die Identifizierung von Komplexbildungen zwischen Proteinen weniger bevorzugt, da Proteine beispielsweise als Wirkstoffe nicht oral verabreicht werden können und häufig zu allergischen Reaktionen führen. Bevorzugt wird beim erfindungsgemäßen Verfahren die Komplexbildung zwischen Proteinen, DNS oder RNS und niedermolekularen Substanzen untersucht. Mindestens einer der Reaktanden kann beim erfindungsgemäßen Verfahren ein Protein oder eine DNS sein; mindestens ein weiterer Reaktand sollte eine niedermolekulare Substanz sein. Auch Wechselwirkungen zwischen lang- und kurzkettigen DNS oder RNS können erfaßt werden.

Vor allem bei der Komplexbildung von und mit Proteinen, aber auch bei vielen anderen organischen Verbindungen, wird vorzugsweise der mittlere IR-Bereich zwischen 2500 und 12500 nm eingesetzt.

Nach einer Wartezeit wird anschließend ein zweites IR- oder FTIR-Spektrum aufgenommen (Zeitpunkt tₙ). Die Wartezeit (= x) bemißt sich nach der Reaktionsgeschwindigkeit der Reaktionspartner. Sie beträgt zwischen 1 ms und einem Tag, bevorzugt zwischen 10 ms und 120 min, besonders bevorzugt zwischen 10 ms und 10 min. Ist ein Reaktand ein Protein, ist eine Wartezeit im Bereich von 5 bis 30 s, beispielsweise 20 s, gut geeignet. Wesentlich kürzere Wartezeiten, etwa im Bereich von 10 bis 100 ms, sind beispielsweise für die Untersuchung des Avidin/Biotin-Komplexes erforderlich. Wartezeiten im Minutenbereich sollten bei der Reaktion mancher Antikörper und bei der Hybridisierung von DNS vorgesehen werden.

Im zweiten IR- oder FTIR-Spektrum wird der bis zu diesem Zeitpunkt erfolgte Reaktionsumsatz dokumentiert. Dieser Reaktionsumsatz läßt sich darstellen, indem aus dem ersten Spektrum, beispielsweise bei t₀, und dem zweiten Spektrum bei tₙ ein Differenzspektrum gebildet wird. Das Differenzspektrum ergibt sich nach ΔAᵥ = ¹⁰log(I₁ᵥ/I₂ᵥ), wobei I₁ᵥ und I₂ᵥ die gemessenen Intensitäten bei der Frequenz v im ersten bzw. zweiten Spektrum sind.

Besteht das Differenzspektrum im wesentlichen aus einer geraden Linie, hat keine Reaktion stattgefunden, da sich in diesem Fall die Konzentration der Reaktionspartner nicht ändert. Eine Bindung der Reaktanden zeigt sich daher in einem Differenzspektrum, das eine Bandenstruktur aufweist.

Unter Komplexen im erfindungsgemäßen Verfahren sind alle kovalenten oder nicht kovalent gebundenen Reaktanden oder deren Teile zu verstehen. Als nicht kovalente Bindungen seien hier beispielhaft Bindungen über van-der-Waals-Kräfte, über Wasserstoffbrückenbildung oder über ionische Bindungen genannt. Erfindungsgemäß werden nicht kovalent gebundene Reaktanden oder deren Teile bevorzugt.

Ein wesentlicher Vorzug des erfindungsgemäßen Verfahren ist, daß der Verfahrensablauf automatisiert werden kann, so daß die Bildung der Reaktandenkomplexe schnell entweder nacheinander oder parallel erfolgen kann. Sogenannte Massenscreening-Verfahren sind insbesondere bei der Entwicklung neuer Arzneimittel von Bedeutung. Hierzu wird ein Protein identifiziert, das mit der Manifestation beispielsweise einer Krankheit in Verbindung steht. Die Aufgabe besteht nun darin, ein geeignetes Arzneimittel zu finden, das dieses sogenannte Target-Protein hemmt. Das Target-Protein kann in großen Mengen und hoher Reinheit aus dem entsprechenden biologischen Material gewonnen werden. Eine Vielzahl von Reagenzien, deren pharmazeutische Wirksamkeit vermutet wird, werden getestet, ob sie die gesuchte Hemmwirkung aufweisen. Die Anzahl der zu testenden Reagenzien ist im allgemeinen sehr groß; sie kann 5-, 6- oder sogar 7-stellig sein, so daß der Einsatz einer schnellen, automatischen Screening-Methode von entscheidender wirtschaftlicher Bedeutung ist.

Diese Aufgabe kann das erfindungsgemäße Verfahren übernehmen. Infolge der hohen Scangeschwindigkeiten moderner FTIR-Spektrometer und dem geringen für das erfindungsgemäße Verfahren erforderlichen Zeitbedarf sind die Voraussetzungen zum Test einer großen Zahl von Reagenzien geschaffen. Ein weiterer wesentlicher Vorteil des Verfahren ist, das problemlos die üblicherweise eingesetzten Standard-Mikrotiterplatten eingesetzt werden können. Da der Trend zu noch kleineren Mikrotiterplatten mit 384 oder sogar 864 Löchern geht, die ein nochmals geringeres Volumen von ca. 100 µl bzw. ca. 50 µl besitzen, ist die geringe erforderliche Probenmenge beim erfindungsgemäßen Verfahren und bei der erfindungsgemäßen Vorrichtung von besonderer Bedeutung. Weitere wichtige Vorteile und die Grundlagen für die Eignung des Verfahrens Massenscreeningverfahren sind, daß kein deuteriertes Wasser eingesetzt werden muß und daß die Vorrichtung ohne konstruktive Änderung für eine Vielzahl von Untersuchungen eingesetzt werden kann.

Nachfolgend wird eine Ausführungsform einer Vorrichtung zur Durchführung des erfindungsgemäßen Verfahrens anhand von Figuren beschrieben.

Es zeigen
Fig. 1 die Ausführungsform der Vorrichtung in einem ersten Betriebszustand;
Fig. 2 die Ausführungsform der Vorrichtung in einem zweiten Betriebszustand;
Fig. 3 die Ausführungsform der Vorrichtung in einem dritten Betriebszustand.

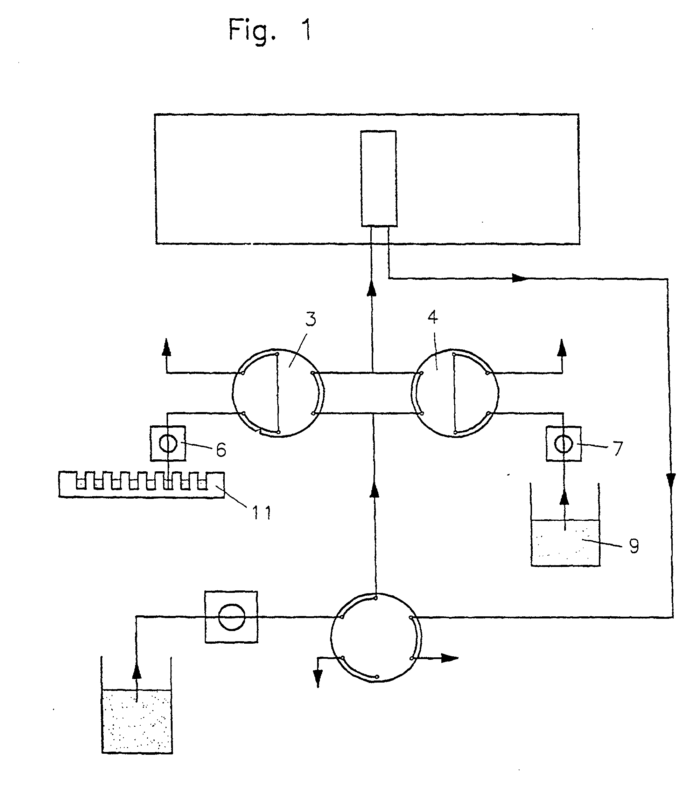

Fig. 1 zeigt die Vorrichtung in einem ersten Betriebszustand, in dem die Ventile 3 und 4 mit einer Testsubstanz bzw. mit einem Targetprotein befülit werden. Die Testsubstanzen werden in einer Standard-Mikrotiterplatte 11 auf einem xyz-Positioniertisch und das Targetprotein in einem Vorratsbehälter 9 vorgelegt. Die Ventile 3 und 4 sind aus der HPLC-Technik bekannte Drehventile (Fa. Valco); sie sind in der dargestellten Position so geschaltet, daß sie mit den beiden Niederdruckpumpen 6 und 7 mit einer Teststubstanz aus der Standard-Mikrotiterplatte 11 bzw. mit dem Targetprotein aus dem Vorratsbehälter 9 befüllt werden können. Ein etwaiger Überschuß wird in beiden Fällen in das Abwassersystem (nicht dargestellt) geleitet.

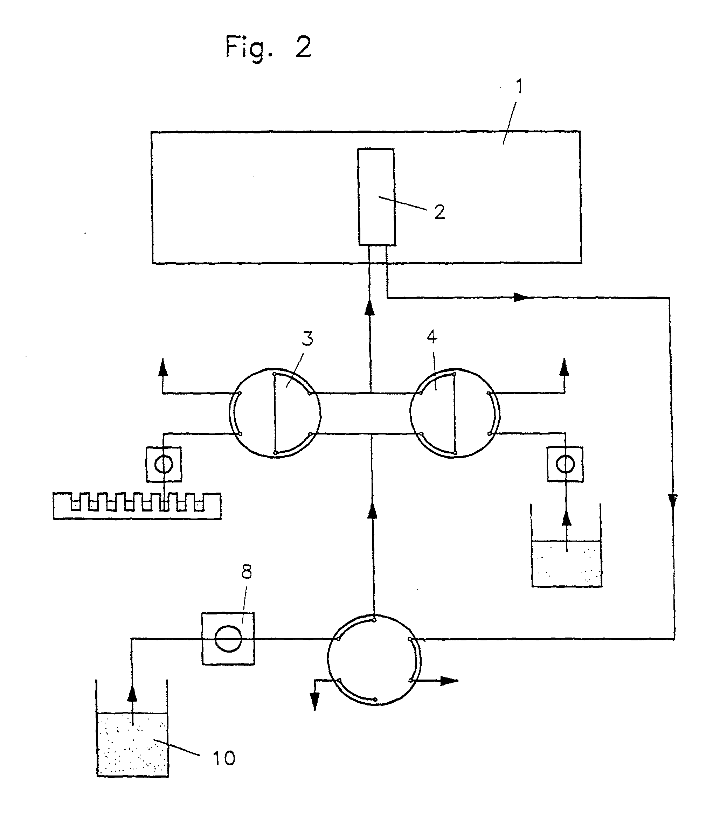

In dem in Fig. 2 dargestellten Betriebszustand sind die Ventile 3 und 4 umgeschaltet. Die Testsubstanz und das Targetprotein werden mit Hilfe der Hochdruckpumpe 8, die an einen Vorratsbehälter 10 für destilliertes Wasser angeschlossen ist, gemischt und in die IR-Küvette 2 im FTIR-Spektralphotometer 1 transportiert.

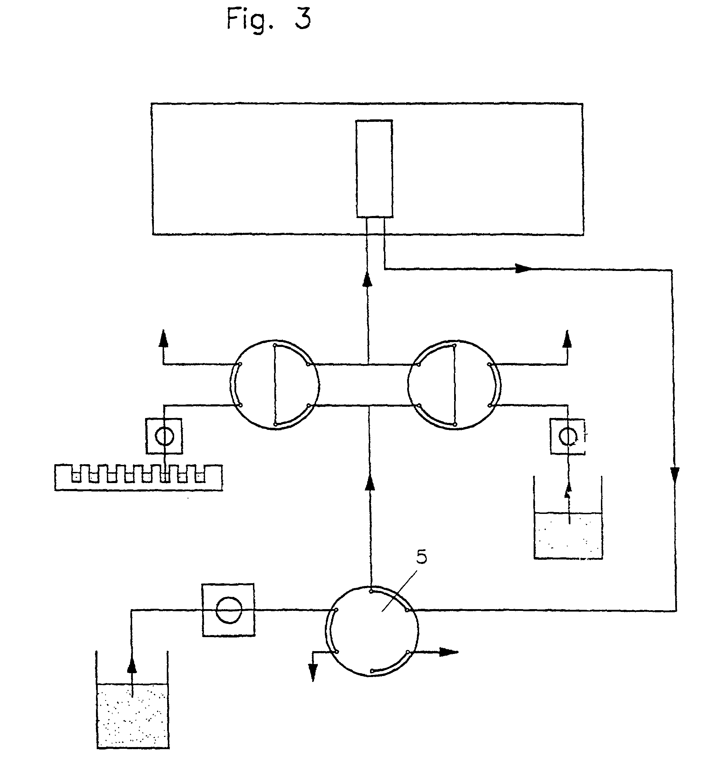

In Fig. 3 ist der Betriebszustand dargestellt, in dem das erste (t₀) und nach der Wartezeit das zweite FTIR-Spektrum (tₙ) aufgenommen werden. Hierbei ist Ventil 5 umgeschaltet, so daß die Testsubstanz und das Targetprotein in einem abgeschlossenen Leitungssystem isoliert sind.

Nach der Aufnahme der Spektren wird die Meßzelle mit destilliertem Wasser gespült, bevor der Zyklus erneut durchlaufen wird.

Die Vorrichtung ist im wesentlichen aus HPLC-Komponenten aufgebaut. Die Hochdruckpumpe 8 ist eine HPLC-Pumpe (Fa. Alltech), die einem kontinuierlichen Hochdruckbetrieb gewachsen ist. Alle Flüssigkeitsleitungen und Verbindungen bestehen aus HPLC-Komponenten, die bis 400 bar belastbar sind. Um die HPLC-Pumpe zu schonen, wird lediglich destilliertes Wasser gepumpt. Die gewählte Anordung der HPLC-Drehventile 3, 4 und 5 bringt die zusätzlichen, für das Massenscreening bedeutsamen Vorteile einer effizienten Reinigung des Systems sowie eines verminderten Probenmaterialverbrauchs. Mit Hilfe des Drehventils 5 wird der Fluß des destillierten Wassers aus dem Vorratsbehälter 10 durch die Hochdruckpumpe 8 umgeleitet anstatt angehalten, um den Fluß durch die IR-Küvette anzuhalten. Dadurch wird die Pumpe geschont und es ergibt sich eine schnellere Antwortzeit. Außerdem werden hierdurch die Eingangs- und Ausgangsleitungen der IR-Küvette kurzgeschlossen, weshalb sich der Überdruck rasch ausgleicht und Schichtdickenänderungen infolge einer Aufwölbung der IR-Küvette beseitigt werden.

Mit der in den Figuren dargestellten Vorrichtung können Durchflußgeschwindigkeiten bis 40 ml/min realisiert werden, was einem Austausch des Inhalts der IR-Küvette innerhalb von 15 ms entspricht. Dabei wird ein Druck von bis zu ca. 150 bar verzeichnet. Das Umleiten des Flüssigkeitsstroms mit Hilfe des Ventils 5 erfolgt in 20 ms. Je 100 µl Targetprotein und Testsubstanz sind für die Aufnahme der FTIR-Spektren ausreichend, wobei durch eine Optimierung der Probenbedarf noch um einen Faktor 3 bis 5 vermindert werden kann.

## Patentansprüche

1. Verfahren zur Identifizierung von Wirkstoffen, bei dem man mindestens zwei einen Komplex bildende Reaktanden miteinander vermischt und das IR-Spektrum der einzelnen im Gemisch noch nicht umgesetzten Reaktanden zu einem ersten Zeitpunkt aufnimmt und mit mindestens einem weiteren IR-Spektrum zu einem zweiten Zeitpunkt den Komplex der Reaktanden erfaßt, ein Differenzspektrum von zwei zu unterschiedlichen Zeitpunkten aufgenommenen IR-Spektren bildet und diejenigen Reaktanden als Wirkstoffe auswählt, deren Differenzspektrum eine Bandenstruktur aufweist.

2. Verfahren nach Anspruch 1, wobei als erster Zeitpunkt der Zeitpunkt unmittelbar nach dem Vermischen der Reaktanden gewählt wird.

3. Verfahren nach Anspruch 1 oder 2, wobei mindestens einer der Reaktanden eine niedermolekulare Verbindung ist.

4. Verfahren nach einem der Ansprüche 1 bis 3, wobei mindestens einer der Reaktanden ein Protein ist.

5. Verfahren nach einem der Ansprüche 1 bis 4, wobei mindestens einer der Reaktanden ein DNS-Molekül ist.

6. Verfahren nach einem der Ansprüche 1 bis 5, wobei zwischen dem ersten und dem zweiten Zeitpunkt eine Wartezeit im Bereich von einer Millisekunde bis zu einem Tag liegt.

7. Verfahren nach einem der Ansprüche 1 bis 6, bei dem das Verfahren nacheinander oder parallel mit einer Vielzahl von Reaktanden durchgeführt wird, wobei die Reaktanden in einer Mikrotiterplatte vorgelegt werden.

8. Verfahren nach einem der Ansprüche 1 bis 7, wobei die Messung der IR-Spektren bei einer Schichtdicke von 1 bis 25 um erfolgt.

9. Vorrichtung zur Durchführung des Verfahrens nach Anspruch 1, bei der
- mit einer ersten Pumpe (6) ein erstes Probenschleifenventil (3) mit einem ersten Reaktanden und
- mit einer zweiten Pumpe (7) ein zweites Probenschleifenventil (4) mit mindestens einem zweiten Reaktanden befüllbar ist,
- die Probenschleifenventile (3, 4) in der Weise miteinander verschaltbar sind, daß sich der erste und der zweite Reaktand in einen abgeschlossenen Raum einschließen lassen,
- der erste und der zweite Reaktand mit einer dritten Pumpe (8) mischen und aus dem abgeschlossenen Raum in eine IR-Küvette (2) fördern lassen,
- Mittel zur Aufnahme von IR-Spektren vorgesehen sind.

## Claims

1. Method of identifying active substances, wherein at least two reactants, which form a complex, are mixed together, and the IR spectrum of the individual reactants, which have not yet reacted in the mixture, is detected at a first point in time, and the complex of the reactants is determined with at least one additional IR spectrum at a second point in time, a differential spectrum of two IR spectra, which were detected at different points in time, is formed, and those reactants, where the differential spectrum has a band structure, are selected as the active substances.

2. Method according to claim 1, wherein the point in time immediately after the mixing of the reactants is selected as the first point in time.

3. Method according to claim 1 or 2, wherein at least one of the reactants is a low-molecular compound.

4. Method according to one of claims 1 to 3, wherein at least one of the reactants is a protein.

5. Method according to one of claims 1 to 4, wherein at least one of the reactants is a DNS molecule.

6. Method according to one of claims 1 to 5, wherein a waiting time in the region of between one millisecond and one day lies between the first point in time and the second point in time.

7. Method according to one of claims 1 to 6, wherein the method is accomplished in succession or parallel with a plurality of reactants, the reactants being displayed in a microtitre plate.

8. Method according to one of claims 1 to 7, wherein the measurement of the IR spectra is effected at a layer thickness of 1 to 25 µm.

9. Apparatus for accomplishing the method according to claim 1, wherein
- a first sample loop valve (3) is fillable with a first reactant by a first pump (6), and
- a second sample loop valve (4) is fillable with at least one second reactant by a second pump (7),
- the sample loop valves (3, 4) are interconnectable in such a manner that the first reactant and the second reactant can be enclosed in a sealed chamber,
- the first reactant and the second reactant can be mixed with a third pump and conveyed from the sealed chamber into an IR cell, and
- means are provided for detecting IR spectra.

## Revendications

1. Procédé d'identification de principes actifs selon lequel on mélange au moins deux composants formant un complexe et on enregistre le spectre IR des différents composants dans le mélange avant leur transformation, à un premier instant et avec au moins un autre spectre IR, on enregistre à un second instant le complexe des composants, on forme un spectre de différence pour les spectres IR enregistrés à des instants différents et on sélectionne comme principes actifs les composants ayant un spectre de différence avec une structure de bande.

2. Procédé selon la revendication 1 selon lequel le premier instant est celui qui suit directement le mélange des composants.

3. Procédé selon les revendications 1 ou 2,
**caractérisé en ce que** l'un des composants a une liaison faiblement moléculaire.

4. Procédé selon l'une quelconque des revendications 1 à 3,
**caractérisé en ce qu'**
au moins l'un des composants est une protéine.

5. Procédé selon l'une quelconque des revendications 1 à 4,
**caractérisé en ce qu'**
au moins l'un des composants est une molécule DNS.

6. Procédé selon l'une quelconque des revendications 1 à 5,
**caractérisé en ce qu'**
entre le premier et le second instants, on a un temps d'attente de l'ordre d'une milliseconde jusqu'à un jour.

7. Procédé selon l'une quelconque des revendications 1 à 6, selon lequel le procédé est exécuté successivement ou en parallèle sur un grand nombre de composants, ceux-ci étant fournis à partir d'une plaque de microtitrage.

8. Procédé selon l'une quelconque des revendications 1 à 7,
**caractérisé en ce que**
la mesure des spectres IR se fait pour une épaisseur de couche de 1 à 25 µm.

9. Dispositif pour la mise en oeuvre du procédé selon la revendication 1 selon lequel :
- avec une première pompe (6), on remplit une première vanne d'échantillonnage (3) avec un premier composant, et
- avec une seconde pompe (7), on remplit une seconde vanne d'échantillonnage (4) avec au moins un second composant,
- les vannes d'échantillonnage (3, 4) sont commutées l'une avec l'autre pour que le premier et le second composants puissent s'enfermer dans un volume fermé,
- le premier et le second composants sont mélangés avec une troisième pompe (8) et à partir du volume fermé, on les transfère dans une cuvette IR (2),
- des moyens pour enregistrer des spectres IR.
